Europäisches Patentamt

European Patent Office

Office européen des brevets

(19)

(11) Publication number: **0 195 691 B1**

# EUROPEAN PATENT SPECIFICATION

(45) Date of publication of patent specification: **06.11.91**

(21) Application number: **86302133.3**

(22) Date of filing: **21.03.86**

(51) Int. Cl.⁵: **C12N 15/17**, C12N 15/81, C07K 7/40, C12P 21/02, C07H 21/04

(54) **Process for the preparation of Insulin Precursors and process for the preparation of human insulin.**

(30) Priority: **22.03.85 DK 1293/85**

(43) Date of publication of application:
**24.09.86 Bulletin 86/39**

(45) Publication of the grant of the patent:
**06.11.91 Bulletin 91/45**

(84) Designated Contracting States:
**AT BE CH DE FR GB IT LI LU NL SE**

(56) References cited:
**EP-A- 0 055 945**
**EP-A- 0 089 007**

(73) Proprietor: **NOVO NORDISK A/S**
**Novo Allé**
**DK-2880 Bagsvaerd(DK)**

(72) Inventor: **Thim, Lars**
**Skiftevej 22**
**DK-2820 Gentofte(DK)**
Inventor: **Norris, Kjeld**
**Ahlmanns Alle 34**
**DK-2900 Hellerup(DK)**
Inventor: **Hansen, Mogens Trier**
**Vinkelvej 21**
**DK-3650 Olstykke(DK)**

(74) Representative: **Brown, John David et al**
**FORRESTER & BOEHMERT Widenmayer-strasse 4/I**
**W-8000 München 22(DE)**

**Description**

BACKGROUND OF THE INVENTION

Human insulin consists of two peptide chains, the A-chain containing 21 amino acid residues and the B-chain containing 30 amino acid residues. The A- and B-chain are joined together by two disulfide bridges connecting the cysteinyl residue at A7 to B7 and A20 to B19, respectively. A third disulphide bridge is formed between the cysteinyl residues A6 and A11.

Human insulin is produced in vivo in the pancreas in the form of preproinsulin. Preproinsulin consists of a prepeptide of 24 amino acid residues followed by proinsulin containing 86 amino acid residues in the configuration: prepeptide-B-Arg-Arg-C-Lys-Arg-Ain which C is the C-peptide of 31 amino acid residues.

During excretion from the islet cells the prepeptide is cleaved off and proinsulin then folds to a structure in which disulfide bridges are formed. The C-peptide is then excised proteolytically to give mature human insulin.

Several attempts have been made to produce insulin, and especially human insulin by means of the recombinant DNA technology. In European patent publication No. 0055945 A the preparation of proinsulin or miniproinsulins from E. coli is described. The process includes expression of a chimeric polypeptide, in vitro cleavage of the chimeric polypeptide, and in vitro formation of disulfide bonds between the A- and B-chain and excision of the bridging chain between the A- and B-chain to give human insulin. In European patent publication No. 68701 the preparation of modified proinsulins with a more or less shortened C-peptide by transformation of E. coli is suggested.

The above methods suffer from several drawbacks mainly stemming from the fact that E. coli is used as transformant organism. The expressed products are not secreted from the cells but accumulate intracellularly in the E. coli host organism. Accumulation of the expressed polypeptide product of the preproinsulin or modified preproinsulin type however enhances the risk of enzymatic degradation of the expressed product. Furthermore, processing, folding and establishing of disulfide bridges must apparently be done in vitro.

A more convenient system for expression of mammalian polypeptides seems to be eukaryote cells and several attempts have been made to express foreign genes in eukaryotes, especially in yeast. Expression of interferon in yeast is described in European patent publication No. 0060057 A and expression and secretion in yeast of proteins heterologous to yeast is described in European patent publication Nos. 0088632A, 0116201 A and 0123544 A.

A method for expressing "pre"-proinsulin in yeast and processing and secretion of the expressed "pre"-proinsulin is described in European patent publication No. 0121884 A. It has, however, been shown by the applicants that insulin precursors of the proinsulin type are sensitive to enzymatic degradation in yeast resulting in very low yields, if any, of secreted proinsulin or mature insulin. In yeast it has been shown that human proinsulin and proinsulin analogous with a more or less shortened C-peptide are particularly sensitive to enzymatic cleavages at the two dibasic sequences flanking the C-peptide region. Apparently these cleavages occur before the establishment of the S-S bridges, resulting in formation of C-peptide, A-chain and B-chain.

THE OBJECT OF THE INVENTION AND SUMMARY THEREOF

The object of the present invention is to generate insulin precursors in high yields in yeast with correctly positioned disulfide bridges between the A- and B-moieties and which may be easily converted into human insulin.

Several insulin precursors of the proinsulin type including proinsulin have been investigated (see the following table 1). DNA-sequences encoding the precursors in question were inserted into a yeast vector system and transformed into yeast according to the technique described above and in the following detailed description. The gene encoding the insulin precursor was provided with a DNA-sequence encoding yeast-recognizable secretion and processing signals fused upstream to the gene for the precursor. In the present study a modified MFαl leader sequence was used in which the segment encoding the four last residues (Glu-Ala-Glu-Ala) of the leader was removed. The modified MFαl leader contains as processing signals a dibasic sequence Lys-Arg which is then fused to the 5'-terminus of the precursor encoding gene. It was found that the leader sequence was cleaved off in all the constructions, i.e. cleavage occurred at the dibasic sequence Lys-Arg upstream to all the insulin precursor genes. However, in all the insulin precursors constructions containing 2 dibasic sequences flanking the C-peptide or a modified C-peptide processing at both dibasic sites before establishment of correctly positioned disulphid bridges was observed and no single chained unprocessed precursor molecule could be isolated from the fermentation broth. Accordingly yeast can not be used as expression system for the production of insulin precursors of the proinsulin type.

Surprisingly it has now been found that when the A- and B-chains of human insulin are linked with only one dibasic sequence (constructions 7-10 in table 1) no cleavage at the dibasic sequence occurs in yeast and single chained insulin precursors with correctly positioned disulphide bridges are obtained in high yields in the fermentation broth from a yeast strain transformed with a DNA-sequence encoding the insulin precursor in question. The present invention seems the more surprising as complete cleavage at Lys-Arg was observed in the leader sequence positioned upstream to the precursor encoding gene.

As insulin precursors of the present type containing a single dibasic sequence between the A- and B-chain can easily be converted into human insulin by in vitro digestion as explained in further details later the present invention provides for an economically attractive process for generating human insulin.

In its first aspect the present invention provides a method for producing insulin precursors of the general formula

B-X-Y-A    (I)

wherein B and A are the B- and A-chains of human insulin crosslinked as in human insulin and wherein X and Y are each lysine or arginine residues in yeast by which method a yeast strain transformed with an expression vehicle comprising a DNA-sequence encoding the insulin precursor is cultivated in a suitable culture medium and the insulin precursor is recovered from the culture medium.

Insulin precursors of the above formula (I) are B-Lys-Lys-A, B-Lys-Arg-A, B-Arg-Lys-A and B-Arg-Arg-A, the former two being preferred.

When cultivating such transformed yeast strains high yields of all of the four insulin precursors were isolated from the culture broth, B-Lys-Lys-A and B-Lys-Arg-A being expressed in the highest yields. Accordingly, from an expression level point of view these two precursors would be the preferred ones.

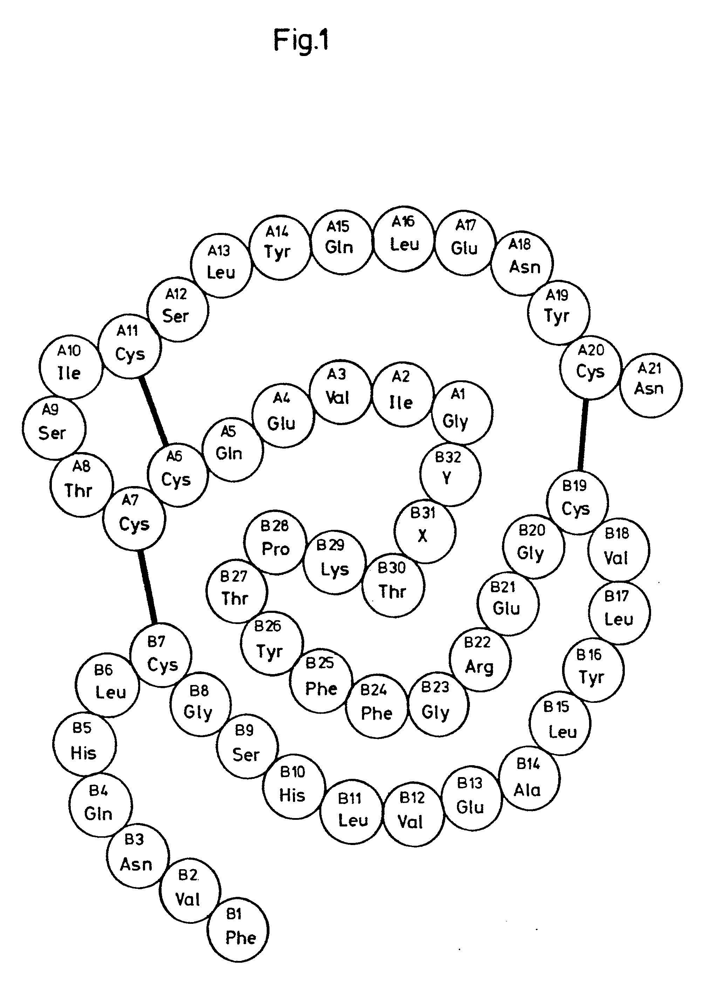

The expression products were isolated and insulin immunoreactive material (IRI-peptides) were purified and characterized by microsequence analysis. It was found that the precursors of the above formula (I) are single chain molecules wherein three disulfide bridges link the following half cystein residues: A6-A11, A7-B7 and A20-B19, i.e. the precursors are expressed in yeast with correctly positioned disulfide bridges compared to human insulin. The structure of the insulin precursors is shown in Fig. 1.

The present invention further provides novel expression vehicles for efficient production of the above insulin precursors in a yeast host and secretion of the precursors into the nutrient medium. The expression vehicles comprise a replication system for stable maintenance in a yeast host, a DNA-sequence encoding the insulin precursors of the above formula (I) and promoter and terminator sequences.

The expression vehicle may upstream to DNA-sequence encoding the desired product contain a preregion ensuring direction of the expressed product into the yeast secretory pathway and secretion of the expressed product into the growth medium. This preregion which might be a naturally occurring signal or leader peptide or a synthetic sequence providing secretion is generally cleaved from the desired product during secretion leaving the mature product ready for isolation from the culture broth.

A well suited leader sequence for yeast is the yeast MFα leader sequence (Kurjan, J. and Herskowitz, I., Cell 30, (1982), 933 - 943).

The expression vehicle may be a plasmid capable of replication in the host microorganism or capable of integration into the host organism chromosome. The vehicle employed may code for expression of repeated sequences of the desired DNA-sequence, each separated by selective cleavage sites.

The expression of the desired DNA-sequence will be under control of a promoter sequence correctly positioned to the DNA-sequence encoding the desired product to result in expression of the desired product in the host organism. Preferably a promoter from a gene indigenous to the yeast host is used, e.g. the promoter of the TPI (triose phosphate isomerase) gene or the MFα-promoter.

The DNA-sequence for the desired product will be followed by a transcription terminator sequence, preferably a terminator sequence from a gene indigenous to the yeast host, e.g. the terminator of the TPI-gene or the MFα-gene.

The present invention also provides novel synthetic DNA-sequences encoding the insulin precursors of the above formular (I). The novel DNA-sequences were constructed by in vitro loop out mutagenesis of the human proinsulin gene using chemically synthesized 30-mer oligonucleotides to delete the original C-peptide encoding sequences.

The present type of insulin precursors can be converted into human insulin by removal of the X and Y amino acid residues by in vitro digestion with trypsin and carboxypeptidase B by a method analogous to the in vitro conversion of proinsulin into insulin described by Kemmler (Kemmler et al., The Journal of

EP 0 195 691 B1

Biological Chemistry, 246 (1971), 6786-6791).

Accordingly, the present invention also provides a method for enzymatically converting the insulin precursors with the above formula (I) into mature human insulin by which method an aqueous solution of the insulin precursors are treated with trypsin and carboxypeptidase B and human insulin is thereafter recovered from the solution.

The enzymatic conversion may be carried out as a one step process by which trypsin and carboxypeptidase B are present at the same time in the reaction mixture. The one step reaction is preferably carried out at about neutral pH and slightly elevated temperatures. The yield of human insulin was about 50%. Better yields were obtained by a two step conversion of insulin precursors of the type B-X-Arg-A. In such two step conversion trypsin is used in the first step, the digested product is isolated and is then further digested with carboxypeptidase B. By carrying out the trypsin digestion at high pH, for instance in the range of 11-12, and at low temperature (about 4°C) the overall yield of human insulin was about 80%. To obtain a high yield in the tryptic digestion at high pH the amino acid residue placed at position B32 (see fig. 1) must be argenine (Y = Arg in formula I) as this residue is still mainly positively charged and accessible to tryptic cleavage. Accordingly, the precursor B-Lys-Arg-A might be the most preferred precursor as it is expressed in high levels in yeast and can be converted into human insulin in very high yields.

Finally the present invention provides a method for preparing human insulin by which a yeast strain transformed with a replicable expression vehicle comprising a DNA-sequence encoding the insulin precursors of the above formula I is cultured in a suitable nutrient medium, and the insulin precursors are recovered from the culture medium and converted into human insulin.

The present invention is not intended to be restricted to the illustrated conversion with trypsin and carboxypeptidase B. If other in vitro enzyme systems with a similar specificity can be found such enzyme systems may be used as well.

BRIEF DESCRIPTION OF THE DRAWINGS

The invention is further illustrated with reference to the accompanying drawings in which
Fig. 1 illustrates the structure of the insulin precursors of formula (I),
Fig. 2 illustrates the preparation of plasmid pMT579,
Fig. 3 illustrates the preparation of plasmid pMT585,
Fig. 4 illustrates the preparation of plasmid pMT644,
Fig. 5 illustrates the preparation of plasmid pMT611,
Fig. 6 illustrates the preparation of plasmid pMT650,
Fig. 7 illustrates the preparation of plasmid pMT658,
and
Fig. 8 illustrates the in vitro conversion of B-Lys-Arg-A into human insulin.

DETAILED DESCRIPTION

1. Preparation of a gene coding for human proinsulin B-C-A

Total RNA purified (Chirgwin, J.M. Przybyla, A.E., McDonald, R.J. & Rutter, W.J., Biochemistry 18, (1979) 5294-5299) from human pancreas was reverse transcribed (Boel, E., Vuust, J., Norris, F., Norris, K., Wind, A., Rehfeld, J.F. & Marcker, K.A., Proc.Natl.Acad.Sci. USA 80, (1983), 2866-2869) with AMV reverse transcriptase and d(GCTTTATTCCATCTCTC) as 1. strand primer. After preparative urea-polyacrylamide gel purification of the human proinsulin cDNA, the second strand was synthesized on this template with DNA polymerase large fragment and d(CAGATCACTGTCC) as 2. strand primer. After S1 nuclease digestion the human proinsulin ds. cDNA was purified by polyacrylamide gel electrophoresis, tailed with terminal transferase and cloned in the PstI site on pBR327 (Sorberon et al., Gene 9, (1980), 287-305) in E. coli. A correct clone harbouring the plasmid was identified from the recombinants by restriction endonuclease analysis and confirmed by nucleotide sequencing (Maxam, A., & Gilbert, W., Methods in Enzymology, 65 - (1980), 499-560. Sanger, F., Nicklen, S. & Coulson, A.R., Proc.Natl.Acad.Sci. USA, 74 (1977), 5463-5467).

The 1. and 2. strand primers, GCTTTATTCCATCTCTC and CAGATCACTGTCC, used for the isolation of a human proinsulin cDNA clone were synthesized by semiautomatic column synthesis using the phophotriester approach on a polystyrene support (H. Ito, Y. Ike, S. Ikata, and K Itakura Nucleic Acids Research 10, (1982), 1755 - 769).

2. Preparation of genes encoding B-X-Y-A

4

Genes encoding the four insulin precursors B-Lys-Lys-A, B-Lys-Arg-A, B-Arg-Lys-A and B-Arg-Arg-A were made by insertion of a fragment encoding the linear human proinsulin sequence B-C-A in circular single stranded M-13 bacteriophage vector and site specific mutagenesis of the human proinsulin sequence with chemically synthesized 30-mer deletion primers, KFN41, KFN4, KFN42 and KFN18, respectively, and an "universal" 15-mer M13 dideoxy sequencing primer (K. Norris et al., Nucl.Acids.Res., 11 (1983), 5103-5112). A double stranded restriction fragment (Xbal-EcoR1) was cut out of the partly double stranded circular DNA and ligated into pUC13 or pT5. By transformation and retransformation of E. coli, transformants harbouring plasmids containing the desired gene were identified.

The four mutagenic deletion primers KFN4, KFN18, KFN41 and KFN42 were synthesized on an automatic DNA synthesizer (Applied Biosystems Model 380 A) using phosphoramidite chemistry and commercially available reagents. (S.L. Beaucage and M.H. Caruthers (1981) Tetrahedron Letters 22, 1859 - 1869). The oligonucleotides were purified by polyacryl amide gel electrophoresis under denaturing conditions. The four deletion primers are as follows:

| KFN | Sequence |
|-----|----------|
| 4 | TCCACAATGCCTCTCTTAGTCTTGGGTGTG |
| 18 | TCCACAATGCCTCTTCTGGTCTTGGGTGTG |
| 41 | TCCACAATGCCCTTCTTGGTCTTGGGTGTG |
| 42 | TCCACAATGCCCTTTCTGGTCTTGGGTGTG |

### 3. Plasmid constructions

Genes encoding the human insulin precursors were combined with fragments coding for the TPI promoter (TPI$_P$) (T. Alber and G. Kawasaki. Nucleotide Sequence of the Triose Phosphate Isomerase Gene of Saccharomyces cerevisiae. J.Mol.Applied Genet. 1 (1982) 419-434), the MFαl leader sequence (J. Kurjan and I. Herskowitz,. Structure of a Yeast Pheromone Gene (MFα): A Putative α-Factor Precursor Contains four Tandem Copies of Mature α-Factor. Cell 30 (1982) 933-943) and the transcription termination sequence from TPI of S. cerevisiae TPI$_T$. These fragments (TPI$_T$) provide sequences to ensure a high rate of transcription for the insulin precursor encoding gene and also provide a presequence which can effect the localization of insulin precursor into the secretory pathway and its eventual excretion into the growth medium.

The expression plasmids further comprise the yeast 2μ origin of replication and a selectable marker LEU2.

During in vivo maturation of α-factor in yeast, the last (C-terminal) six amino acids of the MFα leader peptide (Lys-Arg-Glu-Ala-Glu-Ala) are removed from the α-factor precursor by the sequential action of an endopeptidase recognizing the Lys-Arg sequence and an aminodipeptidase which removes the Glu-Ala residues (Julius, D. et al. Cell 32 (1983) 839-852). To eliminate the need for the yeast aminodipeptidase, the sequence coding for the C-terminal Glu-Ala-Glu-Ala of the MFαl leader was removed via in vitro mutagenesis.

In a preferred construction the modified expression units were transferred to a stable, high copy number yeast plasmid CPOT, (ATCC No. 39685), which can be selected merely by the presence of glucose in the growth medium. Plasmid CPOT is based on the vector Cl/l which has been modified by substituting the original pBR322 Bgl1 - BamH1 fragment with the similar Bgl1 - BamH1 fragment from pUC13 and subsequent insertion of the S.pombe TPI gene (POT) as a BamH1 - Sal1 fragment to give CPOT. Cl/l is derived from pJDB 248, Beggs et al., Nature 275, 104-109 (1978) as described in EP patent application 0103409 A.

### 4. Transformation

Plasmids prepared as described above were transformed into S. cerevisiae strains carrying deletions in the TPI gene by selecting for growth on glucose. Such strains are normally unable to grown on glucose as the sole carbon source and grows very slowly on galactose lactate medium. This defect is due to a mutation in the triose phosphate isomerase gene, obtained by deletion and replacement of a major part of this gene with the S. cerevisiae LEU 2 gene. Because of the growth deficiency there is a strong selection for a plasmid which contains a gene coding for TPI.

5. Expression of the insulin precursors in yeast

The yeast strains containing plasmids encoding different insulin precursors were grown on YPD medium (Sherman, F. et al., Methods in Yeast Genetics, Cold Spring Harbor Laboratory 1981). For each strain, two cultures of 1 litre each in a baffled flask of 2 litres were shaken at 30°C until they reached an OD at 600 nm of approx. 15 (approx. 48 h). After centrifugation the supernatant was removed for further analysis. Immunoreactive insulin (IRI) was measured by radioimmunoassay (Heding, L., Diabetologia 8 (1972), 260-266) by use of semisynthetic human insulin (NOVO Industri A/S) as standard for constructions 1-6. Semisynthetic human insulin or the insulin precursor in question was used for constructions 7-10. For the insulin precursor, B-Arg-Arg-C-peptide-Lys-Arg-A (human proinsulin) the expression level of immunoreactive C-peptide (IRC) was measured by use of a human C-peptide radioimmunoassay (Heding, L.G. Diabetologia 11, (1975) 541-548. In this assay $^{125}$I-Tyr-human-C-peptide was used as tracer and a guinea pig anti-human-C-peptide serum, M1228 (Faber, O.K. et al., Hoppe-Seyler's Z. Physiol.Chem. 357 (1976) 751-757, which reacts equally well with human C-peptide and human proinsulin was used as antibody. Human proinsulin was used as standard (Kruse, V. et al., Diabetologia 27, (1984) 414-415). The expression levels of immunoreactive insulin and immunoreactive C-peptide in the fermentation broth of the transformed yeast strains are summarized in Table 1.

Table 1. EXPRESSION LEVELS OF INSULIN PRECURSORS AS DETERMINED BY RADIOIMMUNOASSAYS.

| Construction No. | Insulin precursor encoding sequence | Yeast strain | Immunoreactive insulin, IRI (µmol/l) | Immunoreactive C-peptide, IRC (µmol/l) |
|---|---|---|---|---|
| 1 | B- Arg-Arg -C-peptide- Lys-Arg -A* | MT-509 | 0.18 | 1.71 |
| 2 | B- Arg-Arg -Glu-Ala-Glu-Asp-Leu-Gln- Lys-Arg -A | ZA-300 | 0.14 | - |
| 3 | B- Arg-Arg -Glu-Ala-Pro-Leu-Gln- Lys-Arg -A | MT-642 | 0.16 | - |
| 4 | B- Arg-Arg -Glu-Ala-Leu-Gln- Lys-Arg -A | MT-641 | 0.17 | - |
| 5 | B- Lys-Arg -Glu-Ala-Leu-Gln- Lys-Arg -A | MT-632 | 0.18 | - |
| 6 | B- Arg-Arg -Leu-Gln- Lys-Arg -A | MT-631 | 0.31 | - |
| 7 | B- Arg-Arg -A | MT-616 (DSM 3195) | 1.00 | - |
| 8 | B- Arg-Lys -A (used in | MT-660 (DSM 3199) | 1.60 | - |
| 9 | B- Lys-Lys -A the invention | MT-655 (DSM 3198) | 2.00 | - |
| 10 | B- Lys-Arg -A | MT-593 (DSM 3194) | 1.70 | - |

*) Identical to human proinsulin encoding sequence.

It appears from table 1 that there is a striking difference in the expression levels of the precursors used in the present invention and precursors of the proinsulin type, i.e. containing a pair of dibasic amino acid flanking the C-peptide or a modified C-peptide.

6. Conversion of the insulin precursors into human insulin

The conversion of the insulin precursors into human insulin can be carried out either by a two step enzymatic process:

$$\boxed{B-X-Y-A} \quad \xrightarrow{\text{Trypsin}} \quad \boxed{B-X-Y \quad A} \qquad (1)$$

$$\boxed{B-X-Y \quad A} \quad \xrightarrow{\text{Carboxypeptidase B}} \quad \boxed{B \qquad A} \qquad (2)$$

or a combined one step process in which trypsin and carboxypeptidase B are present at the same time in the reaction mixture.

In the above formulas and part of the following text the disulfide bridging between the A- and B-chain is illustrated by a bracket

$$(\ "\boxed{\phantom{xxxx}}\ "\ )$$

for better illustration of the enzymatic cleavage process. In the above formulas

$$\boxed{B-X-Y-A}$$

is the one chain insulin precursor (Fig. 1);

$$\boxed{B-X-Y \quad A}$$

is the two chain insulin precursor intermediate in which the peptide bond between residue No. 32 (Lys or Arg) and residue No. 33 (Al = Gly) has been hydrolyzed;

$$\boxed{B \quad A}$$

is human insulin. In the remaining formulas of the type B-X-Y-A it is understood that the A- and the B-chains are connected by disulfide bridges as in native insulin.

The enzymatic conversion is conducted in an aqueous solution of the insulin precursors. The trypsin type is not material to practice of this invention. Trypsin is a well characterized enzyme available in high purity, notably from bovine and porcine pancreas. Enzymes with trypsin-like specificity, e.g. plasmin, clostripain and Achromobacter lyticus protease may also be used.

To obtain a high conversion yield it is important to use highly purified carboxypeptidase B which does not contain carboxypeptidase A activity. This can be achieved by purification of commercially available carboxypeptidase B, e.g. by affinity chromatography or ion-exchange chromatography. Alternatively carboxypeptidase A inhibitors such as $\epsilon$-amino-n-capronic acid or carbobenzoxyglycine can be added to the digest mixture.

The one step conversion is preferably carried out at sligthly elevated temperatures (from 35-40°C) and at a pH of from 7-8. The trypsin and carboxypeptidase B concentration is preferably about 5 $\mu$g/ml and substrate concentration is about 1 mg/ml.

In the two step conversion process the trypsin-digestion is preferably carried out at high pH. At high pH (in the range of 11 - 12) the lysine amino acid residues in the insulin precursor molecule (e.g. B29-Lys and B31-Lys in B-Lys-Arg-A, Fig. 1) are nearly uncharged (pKa for lysine residue is approximately 9.0) and cleavage with trypsin does not occur. However, in e.g.

$$\boxed{B-Lys-Arg-A},$$

the B32-arginine residue (pKa = 12) is still mainly positively charged and thus accessible to tryptic cleavage. Cleavage at the B22-arginine residue does only occur at very low velocity probably due to steric hindrance. A stable pH-value during the tryptic digestion of

$$B-Lys-Arg-A$$

is critical for a high yield of

$$B-Lys-Arg \quad A.$$

Several different buffer systems have been tried with the aim of stabilizing the pH at 11.8-11.9. The buffer system $HPO_4^{--}/PO_4^{---}$ which is normally used at high pH-values has a good buffer capacity at pH 11.8. However, this buffersystem suffers from three main drawbacks. First: the pH of a solution of $HPO_4^{--}/PO_4^{---}$ is very sensitive to temperature variations and a change from e.g. 25°C to 4°C may decrease the pH value with 0.7 pH units. Second: $Ca^{++}$ (which might be present in order to stabilize the trypsin) will be precipitated because the solubility product of $Ca_3(PO_4)_2$ and $CaHPO_4$ is relatively low. Third: it is not possible to acidify the digest mixture (to stop the reaction) momentaneously due to the action of the buffer systems $HPO_4^{--}/H_2PO_4^-$ and $H_2PO_4^{--}/H_3PO_4$. This system will during acidification result in neutral pH for a period of time in which trypsin immediately will create the undesired product des(B30)insulin. As a result of these drawbacks the rather unusual buffer system $H_2O/OH^-$ has been used. This system does not suffer from the above mentioned drawbacks. Optimal conditions for the tryptic digestion of

$$B-X-Arg-A$$

into

$$B-X-Arg \quad A$$

was found to be about 20 mg/ml of

$$B-X-Arg \quad A,$$

200 µg/ml of trypsin; buffer solution: 37.5 mM NaOH (resulting pH as measured at 25°C was 11.86); temperature about 4°C and time of incubation about 180 min. The yield was 90.5% for

$$B-Lys-Arg \quad A.$$

The product of tryptic conversion

$$B-X-Arg \quad A$$

can be purified by a number of conventional methods e.g. preperative HPLC, absorption chromatography, and ion-exchange chromatography.

$$B-X-Arg \quad A$$

can be nearly quantitatively converted into

$$B \quad A$$

(human insulin) by digestion with caboxypeptidase B. Optimal conditions for this conversion was found to be about 5 mg/ml of

$$\text{B-X-Arg} \quad \text{A}$$

and about 5 μg/ml of carboxypeptidase B; buffer solution: 50 mM TRIS HCl, pH = 9.3, temperature about 37° C and time of incubation about 30 min. The yield was 99.5% for the conversion of

$$\text{B-Lys-Arg} \quad \text{A.}$$

The conversion of the insulin precursor

$$\text{B-Lys-Arg} \quad \text{A}$$

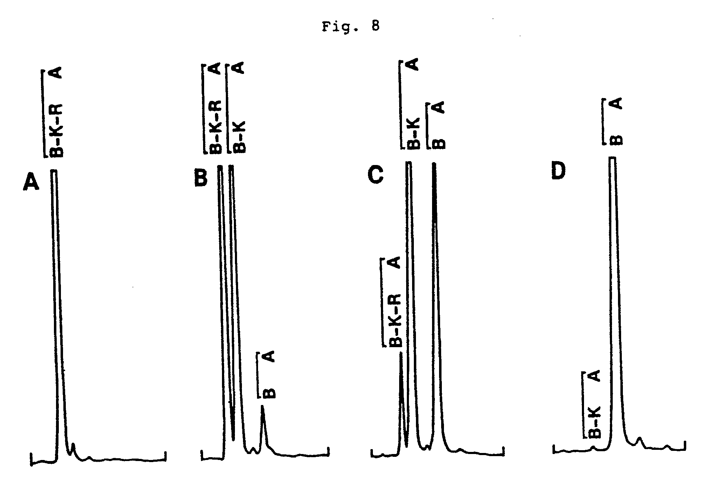

into human insulin was followed quantitatively by reverse phase high pressure liquid chromatography (HPLC) and is illustrated in fig. 8.

Referring to fig. 8,

$$\text{B-Lys-Arg} \quad \text{A}$$

was dissolved to a concentration of 5 mg/ml in 50 mM TRIS-HCl buffer, pH = 9.3 and digested with 5 μg/ml carboxypeptidase B (Boehringer) at 37° C. Aliquots were removed from the digest mixture at t = O- (A), t = 2 min. (B), t = 5 min. (C), and t = 30 min. (D), acidified to pH = 1.5 with 4 N HCl and analysed by HPLC on a 5 μ Nucleosil® RP C-18 column (4 x 200 mm) equilibrated and isocratically eluted with 33 mM $(NH_4)_2SO_4$, 1.5 mM $H_2SO_4$ containing 29.4% (v/v) acetonitrile at 30° C at a flow rate of 1 ml/min. Peptides were detected by UV-absorption at 214 nm. The conversion was completed after 30 min. and ethanol was added to the digest mixture to 60% (v/v).

$$\text{B} \quad \text{A}$$

was purified by anionic exchange chromatography on a QAE-Sephadex® column as described by Schlichtkrull, J. et al., (1974) Horm.Metab.Res.Suppl., Ser. 5, p. 134-143.. B: B-chain, A: A-chain, K: Lys, R: Arg. It appears from fig. 8 that the product from the trypsin digestion

$$\text{B-Lys-Arg} \quad \text{A}$$

was nearly quantitatively converted into

$$\text{B} \quad \text{A}$$

by digestion with carboxypeptidase B.

The conversion of the insulin precursors into human insulin are given in examples 10, 11, and 12 and the characterization of the human insulin in example 13.

EXPERIMENTAL PART

Example 1

Construction of a yeast plasmid pMT585 for the expression of B-Lys-Arg-A.

A 4.3 kb EcoRV-XbaI and a 3.3 kb EcoR1-EcoRV fragment from pMT342 were ligated to a 0.6 kb

EcoR1-XbaI fragment of pM215. Plasmid pMT342 is the yeast vector pMT212 with an inserted TPI$_p$-MFαI leader-BCA-TPI$_T$-sequence. The construction of pMT342 and pMT212 is described in European patent application No. 0068701 A. Plasmid pM215 was constructed by subcloning the EcoR1-Xba1 fragment containing the proinsulin coding sequence B-C-A from p285 (ATCC No. 20681) into pUC13 (constructed as described for pUC8 and pUC9 by Vieira et al., Gene 19: 259-268 (1982)) and subsequent in vitro loop-out removal of the 12 bases coding for Glu-Ala-Glu-Ala at the junction between MFαI leader and proinsulin B-C-A. P285 contains the insert TPI$_p$-MFαI leader-B-C-A-TPI$_T$ and has been deposited in yeast strain Z33 (ATCC No. 20681).

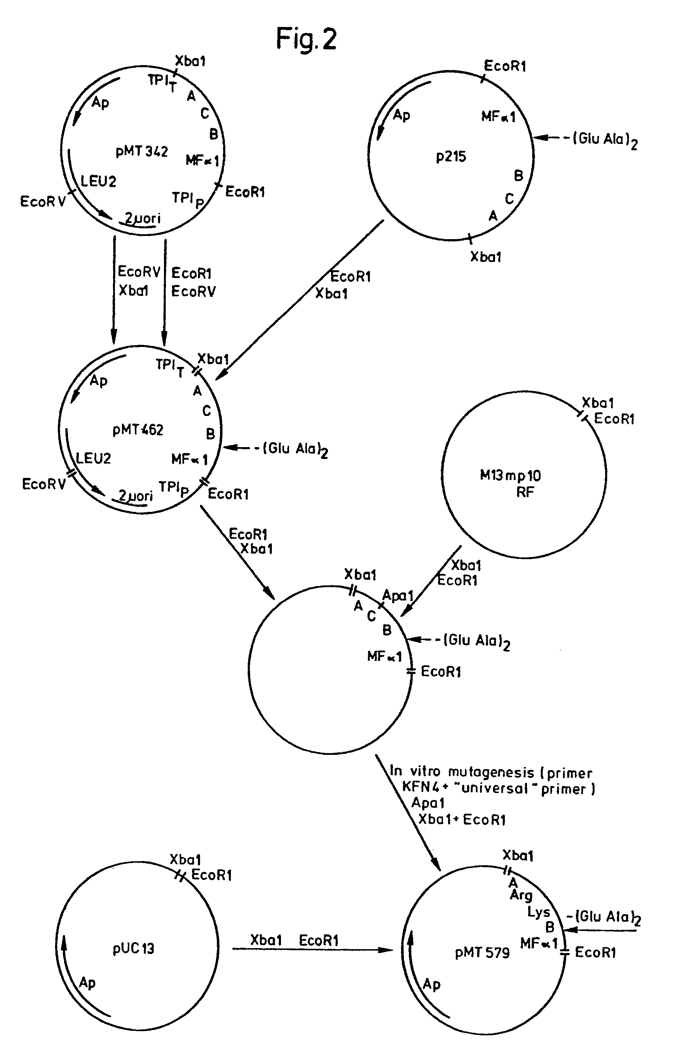

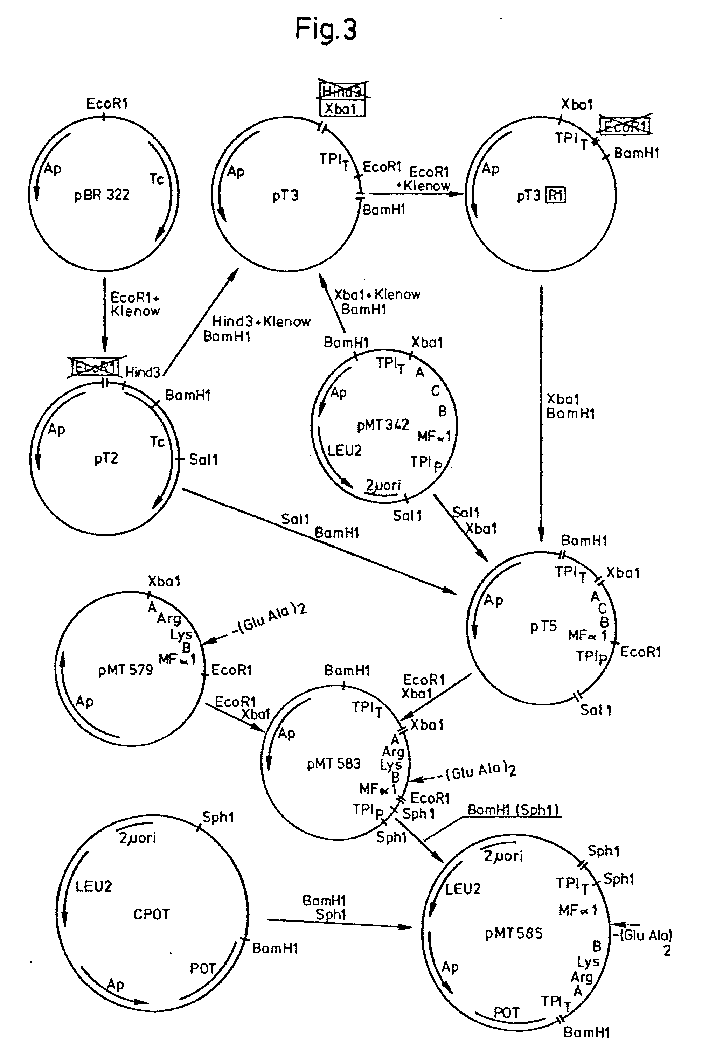

Ligation of the above fragments from pMT342 and pM215 gives plasmid pMT462 harbouring the insert MFαI leader (minus Glu-Ala-Glu-Ala)-B-C-A. For converting the B-C-A encoding fragment into a B-Lys-Arg-A encoding fragment the modified site specific mutagenesis procedure (K. Norris et al., ibid.) was used. A 0.6 kb EcoR1-Xba1 fragment from pMT462 encoding MFαI leader-(minus Glu-Ala-Glu-Ala)-B-C-A was inserted into phage M13 mp10 RF (Messing, J. and Vieria, J. (1983) Unpublished results) DNA cut with Xba1-EcoR1. Single strand M13 phage containing the above EcoR1-Xba1 insert was incubated with the 30-mer deletion primer KFN4 and the "universal" 15-mer M13 primer d(TCCCAGTCACGACGT) (New England Biolabs), heated to 90°C for 5 minutes and slowly cooled to room temperature in order to allow annealing. Then partly double stranded DNA was made by addition of a d-NTP-mix, Klenow Polymerase and T4 ligase. After phenol extraction, ethanol precipitation and resuspension, the DNA was cut with restriction enzymes Apa1, Xba1 and EcoR1. After another phenol extraction, ethanol precipitation and resuspension, the DNA was ligated to EcoR1-Xba1 cut pUC13. The ligation mixture was transformed into an E. coli (r⁻m⁺) strain and plasmids were prepared from a number of transformants. Plasmid preparations were cut with EcoR1 and Xba1 and those preparations showing bands at both 0.5 and 0.6 kb were retransformed into E. coli. From the retransformation a transformant harbouring only pUC13 with a 0.5 kb insert was selected. The sequence of the EcoR1-Xba1 insert of this plasmid, pMT579, was then confirmed by the Maxam-Gilbert method to encode MFαI leader-(minus Glu-Ala-Glu-Ala)-B-Lys-Arg-A. The construction of pMT579 is shown in Fig. 2. The XbaI-EcoR1 insert from pMT579 was provided with TPI promotor and TPI terminator by ligation of a 0.5 kb Xba1-EcoR1 fragment of pMT579 with a 5.5 kb Xba1-EcoR1 fragment of pT5. The construction of pT5 harbouring the insert TPI$_p$-MFαI leader-B-C-A-TPI$_T$ is illustrated in Fig. 3. The resulting plasmid pMT583 containing the insert TPI$_p$-MFαI leader-(minus Glu-Ala-Glu-Ala)-B-Lys-Arg-A-TPI$_T$ was then cut with BamH1 and partially with Sph1 and the 2.1 kb fragment was inserted in CPOT cut with BamH1 and Sph1. The resulting plasmid pMT585 was used for transformation of yeast. The construction of pMT583 and pMT585 is shown in Fig. 3.

## Example 2

### Construction of a yeast plasmid pMT611 for expression of B-Arg-Arg-A

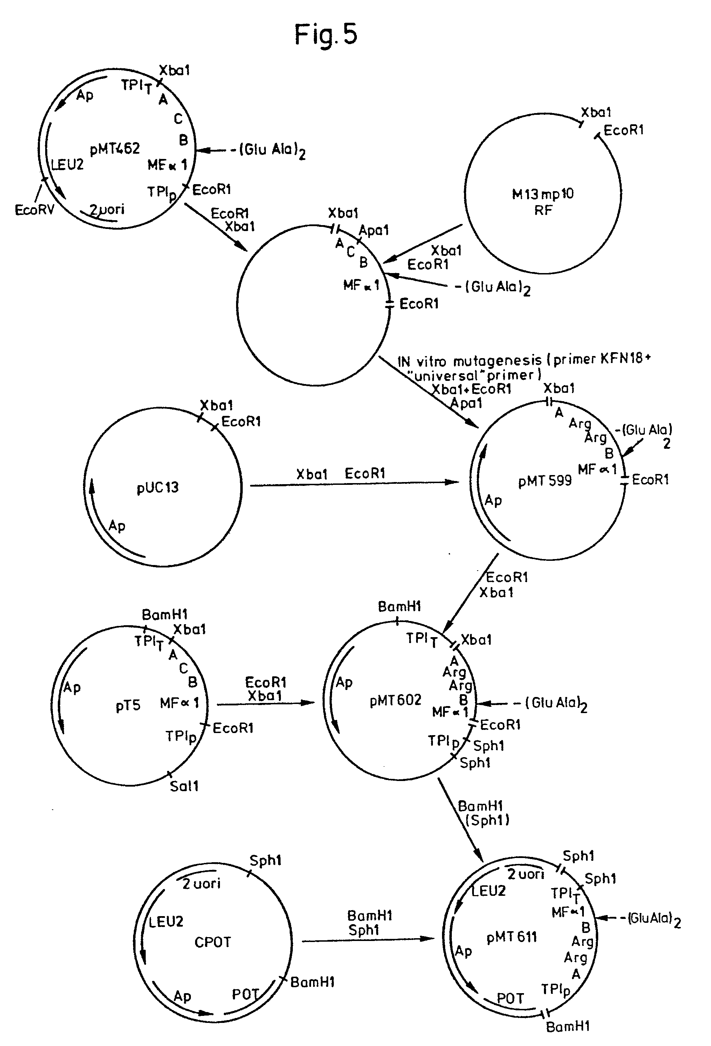

The B-C-A encoding fragment from pMT462 (see example 1) was converted into B-Arg-Arg-A by a procedure analogous with the procedure described in example 1 by site specific mutagenesis with a mixture of the 30mer deletion primer KFN18 and the "universal" 15-mer M13 primer as illustrated in Fig. 5. The sequence of the EcoR1-Xba1 insert of plasmid pMT599 was confirmed by the Maxam-Gilbert method to encode MFαI leader-(minus Glu-Ala-Glu-Ala)-B-Arg-Arg-A. The XbaI-EcoR1 insert from pMT599 was provided with the TPI promoter and TPI terminator by ligation of a 0.5 kb XbaI-EcoR1 fragment of pMT599 with a 5.5 kb XbaI-EcoR1 fragment of pT5. The resulting plasmid pMT602 containing the insert TPI$_p$-MFαI leader-(minus Glu-Ala-Glu-Ala)-B-Arg-Arg-A-TPI$_T$ was then cut with BamH1 and partially with Sph1 and the 2.1 kb fragment was inserted in CPOT cut with BamH1 and Sph1. The resulting plasmid pMT611 was used for transformation of yeast. The construction of plasmid pMT611 is shown in Fig. 5.

## Example 3

### Construction of a yeast plasmid pMT650 for the expression of B-Lys-Lys-A

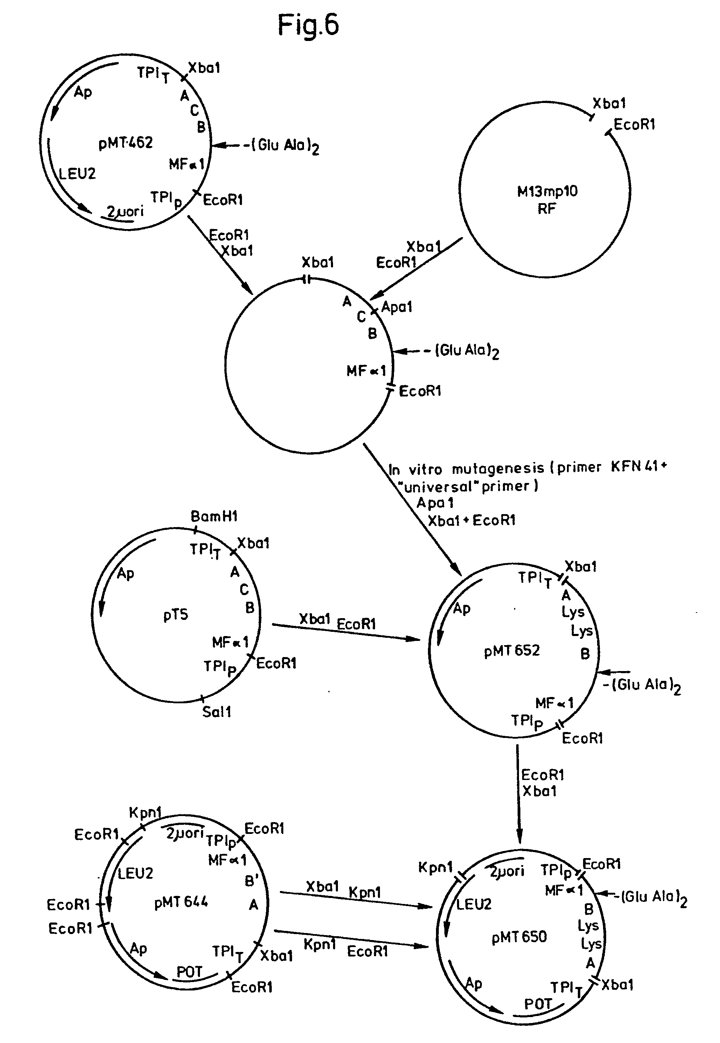

The B-C-A encoding fragment from pMT462 (see example 1) was converted into B-Lys-Lys-A by a procedure similar to the procedure described in example 1 by site specific mutagenesis with a mixture of the 30mer deletion primer KFN41 and the "universal" 15-mer M13 primer as illustrated in Fig. 6.

After filling in with Klenow polymerase and ligation with T4 ligase the partly double stranded DNA was digested with Apa1, EcoR1 and Xba1 and ligated with the 5.5 kb Xba1-EcoR1 fragment from plasmid pT5 (see example 1). After transformation and retransformation into E. coli, a plasmid pMT652 containing the

insert MFαI leader-(minus Glu-Ala-Glu-Ala)B-Lys-Lys-A was isolated and the sequence of the insert confirmed as described above.

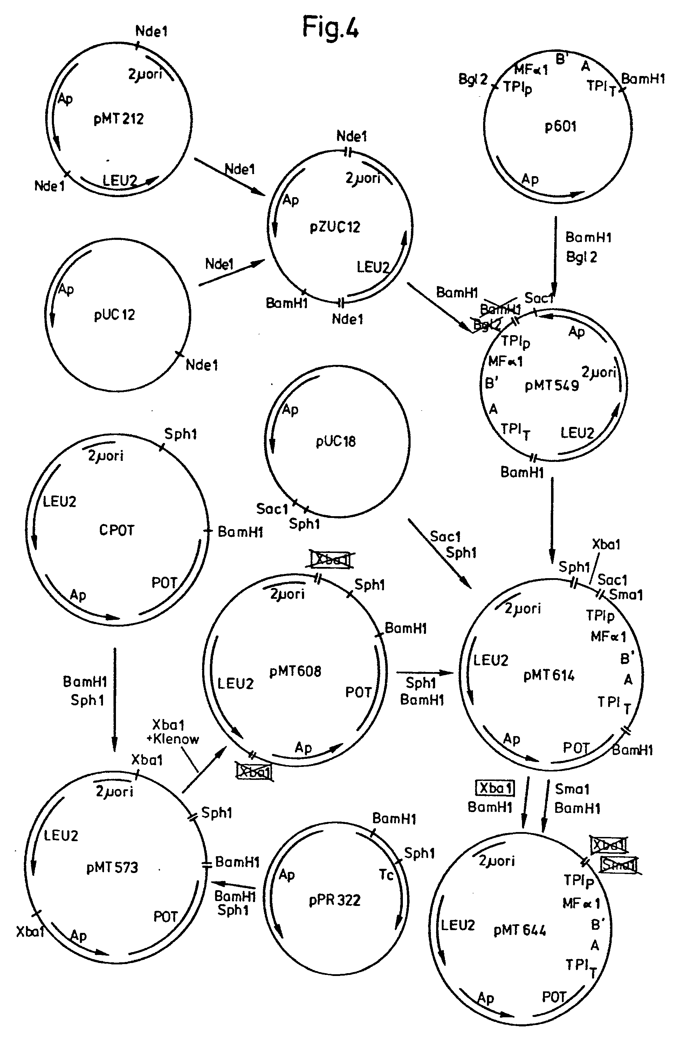

Plasmid pMT652 was cleaved with Xba1-EcoR1 and the 0.5 kb fragment was ligated with a 7.8 kb Xba1-Kpn1 and a 4.3 kb Kpn1-EcoR1 fragment of pMT644. The resulting plasmid pMT650 contains the insert $TPI_p$-MFαI leader (minus Glu-Ala-Glu-Ala)-B-Lys-Lys-A-$TPI_T$ and furthermore contains the TPI coding gene (POT) from CPOT. The construction of plasmid pMT650 is illustrated in Fig. 6 and the construction of pMT644 is shown in Fig. 4. Plasmids pUC12 and pUC18 used for the construction of pMT644 were constructed as described for pUC13 (Vieira et al.. ibid.) Plasmid p601 (see Fig. 4) contains the insert $TPI_p$-MFαI leader -B'A-$TPI_T$ with flanking Bgl2 and a BamH1 sites. B'A stands for B(1-29)-A(1-21), where B(1-29) is a shortened B-chain of human insulin from $Phe^{B1}$ to $Lys^{B29}$ and A(1-21) is the A-chain of human insulin. The construction of a DNA-sequence encoding B'A is described in European patent application No. 0068701 A. pMT650 was used for transformation of yeast.

Example 4

Construction of plasmid pMT658 for the expression of B-Arg-Lys-A

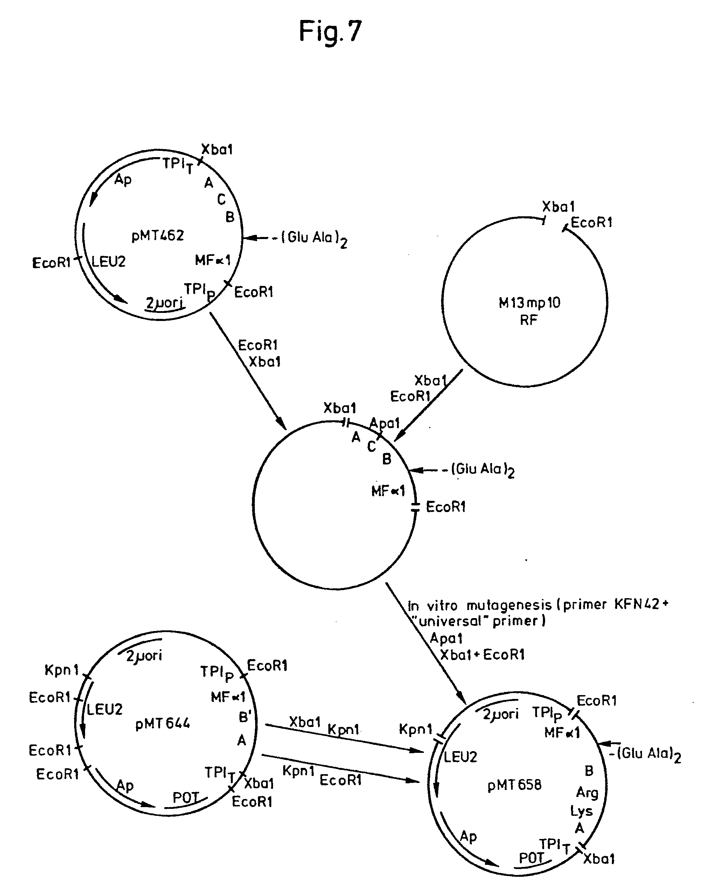

Plasmid pMT658 was constructed as described for pMT650 in example 3 with the exception that the 30mer deletion primer KFN42 was used instead of KFN41. Also, a more direct construction of the pMT644 derivative was chosen: after in vitro mutagenesis, i.e. after filling in with Klenow polymerase and ligation with T4 ligase, the partly double stranded DNA was digested with Apa1, EcoR1 and Xba1 and ligated to a 7.8 kb Xba1-Kpn1 and a 4.3 kb Kpn1-EcoR1 fragment of pMT644. The ligation mixture was transformed into E. coli ($r^-$ $m^+$) and plasmid prepared from a number of transformants. One plasmid preparation showing Xba1-EcoR1 fragments at both 0.6 and 0.5 kb was retransformed into E. coli to give a strain harbouring a plasmid pMT658 containing the 0.5 kb but not the 0.6 kb EcoR1-Xba1 fragment. pMT658 contains the insert $TPI_p$-MFαI leader-(minus Glu-Ala-Glu-Ala)-B-Arg-Lys-A-$TPI_T$. The sequence of the B-Arg-Lys-A coding segment was verified by Maxam-Gilbert DNA-sequencing. The construction of pMT658 is illustrated in fig. 7. pMT658 was used for transformation of yeast.

Example 5

Transformation

S. cerevisiae strain MT501 (E2-7B X E11-3C a/α,Δ tpi/tpi,Δpep 4-3/pep 4-3) was grown on YPGaL (1% Bacto yeast extract, 2% Bacto peptone, 2% galactose, 1% lactate) to an $OD_{600nm}$ of 0.6.

100 ml of culture was harvested by centrifugation, washed with 10 ml of water, recentrifuged and resuspended in 10 ml of 1.2 M sorbitol, 25 mM $Na_2EDTA$ pH = 8.0, 6.7 mg/ml dithiotreitol. The suspension was incubated at 30°C for 15 minutes, centrifuged and the cells resuspended in 10 ml of 1.2 M sorbitol, 10 mM $Na_2EDTA$, 0.1 M sodium citrate pH = 5.8, 2 mg Novozym® 234. The suspension was incubated at 30°C for 30 minutes, the cells collected by centrifugation, washed in 10 ml of 1.2 M sorbitol and in 10 ml of CAS (1.2 M sorbitol, 10 mM $CaCl_2$, 10 mM Tris (Tris = Tris(hydroxymethyl)-aminometan) pH = 7.5) and resuspended in 2 ml of CAS. For transformation 0.1 ml of CAS-resuspended cells were mixed with approximately 1 μg of plasmid pMT585 and left at room temperature for 15 minutes. 1 ml of 20% polyethylenglycol 4000, 10 mM $CaCl_2$, 10 mM Tris pH = 7.5 was added and the mixture left for further 30 minutes at room temperature. The mixture was centrifuged and the pellet resuspended in 0.1 ml of SOS (1.2 M sorbitol, 33% v/v YPGaL, 6.7 mM $CaCl_2$, 14 μg/ml leucine) and incubated at 30°C for 2 hours. The suspension was then centrifuged and the pellet resuspended in 0.5 ml of 1.2 M sorbitol. 6 ml of top agar (the SC medium of Sherman et al., (Methods in Yeast Genetics, Cold Spring Harbor Laboratory, 1981) with leucine omitted and containing 1.2 M sorbitol plus 2.5% agar) at 52°C was added and the suspension poured on top of plates containing the same agar-solidified, sorbitol containing medium. Transformant colonies were picked after 3 days at 30°C, reisolated and used to start liquid cultures. One such transformant MT593 (= MT501/pMT585) was chosen for further characterization.

Plasmids pMT611, pMT650 and pMT658 were transformed into S. cerevisiae strain MT501 by the same procedure as above and the transformants MT616 (= MT501/pMT611), MT655 (= MT501/pMT650) and MT660 (= MT501/ pMT658) were isolated.

The transformed microorganisms MT 593, MT 616, MT 655 and MT660 were deposited by the applicant with Deutsche Sammlung von Mikroorganismen (DSM), Griesebachstrasse 8, D-3400 Göttingen, on January 16, 1985 and accorded the reference numbers DSM 3194, DSM 3195, DSM 3198, and DSM 3199, respectively. DSM being an international depository authorized under the Budapest Treaty of 1977 affords permanence of the above deposits and accessibility thereto by the public in accordance with Rules 9 and 11, respectively, of the above treaty.

Example 6

Purification of B-Lys-Arg-A from yeast strain MT 593 (DSM 3194)

Yeast strain MT 593 (DSM 3194) was grown on YPD medium. A one liter culture in a 2 liter baffled flask was shaken at $30^{\circ}$C to an $OD_{600nm}$ of 15. After centrifugation expression products from 815 ml of supernatant were isolated as follows:

A column (1.5 x 9 cm) of LiChroprep® RP-18 (Merck, art. 9303) was washed with 30 ml of 50 mM $NH_4HCO_3$ containing 60% (v/v) ethanol. The column was equilibrated with 50 ml of 50 mM $NH_4HCO_3$.95 ml of 96% ethanol were added to 815 ml of the yeast supernatant and the mixture was pumped through the column over night (flow 45 ml/h).

The column was washed with 15 ml of 0.1 M NaCl and then with 15 ml of $H_2O$ and peptide material was eluted with 50 mM $NH_4HCO_3$ containing 60% (v/v) of ethanol. The eluate (4.5 ml) was concentrated to 1.1 ml by vacuum centrifugation (Savant vacuumcentrifuge) in order to remove the ethanol, and the volume was adjusted to 10 ml with 25 mM HEPES buffer at pH = 7.4. The sample was applied to an antiinsulin sepharose column (2.5 x 4.5 cm) which prior to the application had been washed with 20 ml of NaFAM buffer (Heding, L., Diabetologia 8 (1972), 260-66) and with 10 ml of 25 mM HEPES buffer pH = 7.4. After the application the column was allowed to stand for 30 min. at room temperature and thereafter washed with 40 ml of 25 mM HEPES buffer, pH = 7.4. The peptide material was eluted with 20% acetic acid and the pH of the eluate was adjusted to 7.0 with $NH_4OH$.

The eluate from the previous step was concentrated to 250 $\mu l$ by vacuum rotation and peptides were further purified by reverse phase HPLC on a 5$\mu$ Waters Novapak C-18 column (3.9 x 150 mm). The A and B buffers were 0.1% TFA in $H_2O$ and 0.07% TFA in acetonitrile, respectively. The column was equilibrated with 25% B at a flow of 0.75 ml/min and the peptides were eluted with a linear gradient (1% acetonitrile per min) and detected at 276 nm. The main peak eluted with a retention time of 13.15 minutes and peptide material from this peak was isolated by lyophilization of the eluate. The peptide material was characterized as described in example 8. The yield in each step of the purification was determined by radioimmunoassay as previously described, and Table 2 summarizes the purification. The overall yield was 39%.

Table 2

| Purification of B-Lys-Arg-A from yeast strain MT 593 | | |
|---|---|---|
| Purification step | Volume (ml) | Immunoreactive B-Lys-Arg-A (nmol) |
| Supernatant | 815 | 813 |
| RP-18 column | 10 | 620 |
| Anti-insulin sepharose column | 4 | 450 |
| Preparative HPLC | 2 | 320 |

Example 7

Purification of B-Lys-Lys-A, B-Arg-Lys-A and B-Arg-Arg-A from yeast strains MT 655 (DSM 3198), MT 660 (DSM 3199) and MT 616 (DSM 3195), respectively

The above mentioned yeast strains were grown as previously described in example 6 and expression products were purified from the different supernants essentially as described in example 6. Table 3 summarizes the overall yields.

Table 3

| Yeast strain | Supernatant volume (ml) | Peptide yield (nmol) | Overall yield (%) |
|---|---|---|---|
| MT 655 (DSM 3198) | 750 ml | 689 | 53 |
| MT 660 (DSM 3199) | 500 ml | 395 | 38 |
| MT 616 (DSM 3195) | 600 ml | 301 | 49 |

Example 8

Characterization of B-Lys-Arg-A purified from yeast strain MT 593 (DSM 3194)

B-Lys-Arg-A was purified as described in example 6. The amino acid composition of the peptide was determined as follows: 139.6 µg (19 nmol) was hydrolysed in 100 µl 6 N HCl for 24 h at 110°C. The hydrolysate was analyzed on a Beckman Model 121 M amino acid analyser. The following amino acid composition was found:

Table 4

| Amino acid analysis of purified B-Lys-Arg-A | | | | | |
|---|---|---|---|---|---|
| Amino acid | Found | Theory | Amino acid | Found | Theory |
| Asx* | 2.92 | 3 | Val | 3.71 | 4 |
| Thr | 2.77 | 3 | Ile | 1.62 | 2 |
| Ser | 2.59 | 3 | Leu* | 6.03 | 6 |
| Glx* | 7.01 | 7 | Tyr | 3.83 | 4 |
| Pro | 1.37 | 1 | Phe* | 2.93 | 3 |
| Gly* | 3.95 | 4 | Lys* | 1.96 | 2 |
| Ala* | 1.03 | 1 | His* | 2.01 | 2 |
| 1/2 Cys | 5.47 | 6 | Arg* | 2.08 | 2 |
| *) Amino acid used for normalization | | | | | |

Approximately 5 nmol peptide material was subjected to amino acid sequence analysis. The sequence analysis was performed with a Gas Phase Sequencer (Applied Biosystem Model 470 A) as descbribed by Moody, A.J., Thim, L. and Valverde, I. (FEBS Lett., 172 (1984), 142-148). The following results were found:

## EP 0 195 691 B1

Table 5
Amino acid sequence analysis of purified B-Lys-Arg-A

| Cycles | PTH-amino acid residue | | Yield (pmol) |
|---|---|---|---|
| 1 | Phe | B1 | 1700 |
| 2 | Val | | 977 |
| 3 | Ash | | 2100 |
| 4 | Gln | | 951 |
| 5 | His | | 1327 |
| 6 | Leu | | 1717 |
| 7 | Cys | | – |
| 8 | Gly | | 938 |
| 9 | Ser | | 184 |
| 10 | His | | 591 |
| 11 | Leu | | 718 |
| 12 | Val | | 777 |
| 13 | Glu | | 363 |
| 14 | Ala | | 491 |
| 15 | Leu | | 408 |
| 16 | Tyr | | 723 |
| 17 | Leu | | 724 |
| 18 | Val | | 631 |
| 19 | Cys | | – |
| 20 | Gly | | 187 |
| 21 | Glu | | 239 |
| 22 | Arg | | 413 |
| 23 | Gly | | 240 |
| 24 | Phe | | 456 |
| 25 | Phe | | 425 |
| 26 | Tyr | | 223 |
| 27 | Thr | | 78 |
| 28 | Pro | | 109 |
| 29 | Lys | | 158 |
| 30 | Thr | B30 | 59 |
| 31 | Lys | | 94 |
| 32 | Arg | | 58 |
| 33 | Gly | A1 | 56 |
| 34 | Ile | | 179 |
| 35 | Val | | 103 |
| 36 | Glu | | 83 |
| 37 | Gln | | 136 |
| 38 | Cys | | – |
| 39 | Cys | | – |
| 40 | Thr | | 17 |
| 41 | Ser | | trace |
| 42 | Ile | | 53 |
| 43 | Cys | | – |
| 44 | Ser | | trace |
| 45 | Leu | | 60 |
| 46 | Tyr | | 43 |
| 47 | Gln | | trace |
| 48 | Leu | | 64 |
| 49 | Glu | | 40 |
| 50 | Asn | | 29 |
| 51 | Tyr | | trace |
| 52 | Cys | | – |
| 53 | Asn | A21 | 30 |

The average repetitive yield was 92.1%.

## Example 9

Characterization of B-Lys-Lys-A, B-Arg-Lys-A and B-Arg-Arg-A purified from yeast strains MT 655 (DSM 3198), MT 660 (DSM 3199) and MT 616 (DSM 3195), respectively.

Peptide material was purified from the above mentioned yeast strains as described in example 6. The peptides were submitted to amino acid sequence analysis as described in example 8. From the sequence results (not shown) it could be concluded that the dibasic sequences linking the B- and A-chain were Lys-Lys (MT 655), Arg-Lys (MT 660) and Arg-Arg (MT 616), respectively. Purified peptides were submitted to amino acid analysis as described in example 7. The amino acid compositions were found in accordance with the theory (results shown only for B-Lys-Lys-A).

## Table 6

| Amino acid analysis of purified B-Lys-Lys-A | | | | | |
|---|---|---|---|---|---|
| Amino acid | Found | Theory | Amino acid | Found | Theory |
| Asx* | 2.95 | 3 | Val | 3.74 | 4 |
| Thr | 2.77 | 3 | Ile | 1.68 | 2 |
| Ser | 2.59 | 3 | Leu* | 6.05 | 6 |
| Glx* | 6.92 | 7 | Tyr | 3.84 | 4 |
| Pro | 1.14 | 1 | Phe* | 2.97 | 3 |
| Gly* | 3.95 | 4 | Lys* | 2.94 | 3 |
| Ala* | 1.03 | 1 | His* | 1.96 | 2 |
| 1/2 Cys | 5.44 | 6 | Arg* | 1.05 | 1 |
| *) Amino acid used for normalization | | | | | |

Example 10

Conversion of B-Lys-Arg-A into human insulin (one step process)

10 mg B-Lys-Arg-A was dissolved in 10 ml of 0.23 M TRIS-HCl buffer pH = 7.5. The solution was heated to 37°C and 50 $\mu$g trypsin (NOVO Industri A/S) and 50$\mu$g carboxypeptidase B (Boehringer) both dissolved in 100 $\mu$l of water was added to time zero. Aliquots of the reaction mixture were removed to time 0 min., 2 min., 10 min., 40 min., and 80 min. The enzyme reaction was stopped by acidification of the samples to pH = 2.5 with 1 M HCl.

The conversion of B-Lys-Arg-A into human insulin was followed by reverse phase HPLC on a 5 $\mu$ RP C-18 column (4 x 200 mm) of Nucleosil (Macherey Nagel). The A buffer consisted of 25% acetonitrile adjusted to pH = 3.5 with $H_2SO_4$ and the B buffer was 45% acetonitrile, 0.15 M $(NH_4)_2SO_4$, 1.5 mM $H_2SO_4$. The eluation was performed in an isocratic system using a mixture of 80% A-buffer/20% B-buffer at a flow of 1 ml/min. The temperature of the solvents and column was 30°C and peptides were detected at 214 nm.

The optimal yield in the above one step process was obtained after 10 min of incubation. At this time the reaction mixture consisted of: 45% human insulin, 10% Des-B30-insulin and 45% Arg-Ao-insulin. In order to increase the yield of the conversion a two step process was designed (Example 11).

Example 11

Conversion of B-Lys-Arg-A into human insulin (two step process)

26.4 ml of $H_2O$ was added to 471 mg

$$\overline{\text{B-Lys-Arg-A}} \; .$$

The mixture was chilled to 4°C and 3.0 ml of 0.25 M NaOH was added whereby the insulin precursor was dissolved. 6 mg of trypsin (NOVO Industri A/S) in 200 µl of water was added. The reaction was carried out at 4°C for 5 h and stopped by the addition of 600 µl of 4 N HCl. The yield as determined by HPLC (method given in Example 10) was 91.5% of

$$\overline{\text{B-Lys-Arg} \quad \text{A}}$$

and the main part of the remaining 8.5% was undigested

$$\overline{\text{B-Lys-Arg-A}}.$$

The product

$$(\overline{\text{B-Lys-Arg} \quad \text{A}})$$

was purified by preparative HPLC on a column (5 x 25 cm) of octadecyldimethylsilyl substituted silica (average particle size: 15 µ, pore size: 100 Å). The column was equilibrated and eluted (isocratic) with 0.185 M KCl/0.6 mM HCl (pH = 3.15) containing 37% (v/v) ethanol at a flow rate of 2 l/h. B-Lys-Arg A eluted at 4.3 column volumes and was isolated from the alcoholic pool by a crystallization procedure previously described for crystallization of human insulin B-30 esters (Markussen, J. (1984) in "Diabetes Research Vol I, Laboratory Methods Part B" p. 403-411, eds. Larner and Pohl).

The crystals were lyophilized and dissolved in 50 mM TRIS-HCl buffer (pH = 9.3) in a concentration of 10 mg/ml. The insulin precursor intermediate was converted into human insulin by digestion with carboxypeptidase B (40 µg/ml) at 37°C for 40 h. To the digest mixture was then added ethanol to a final concentration of 60% (v/v) and human insulin was purified from the mixture by anionic exchange chromotography on a QAE-Sephadex (Pharmacia) column as described by Schlichtkrull et al. (Horm.Metab.Res.Suppl., Ser. 5 (1974) 134-143).

The yields at the different steps of the conversion including the purifications are given in Table 7.

## Table 7

| Conversion of B-Lys-Arg-A into human insulin | | | |
|---|---|---|---|
| Step | Product | Amount (mg) | Yield (%) |
| Starting material | ⌐¬ B-Lys-Arg-A | 471 | 100 |
| Tryptic digest | ⌐¬ B-Lys-Arg A | 431 | 91.5 |
| HPLC-pool | ⌐¬ B-Lys-Arg A | 399 | 84.7 |
| Carboxypeptidase-B digest | ⌐ B A (human insulin) | 379 | 80.5 |
| QAE-pool | ⌐ B A (human insulin) | 359 | 76.2 |

Example 12

Conversion of B-Lys-Lys-A into human insulin (one step process)

10 mg of B-Lys-Lys-A was converted to human insulin as described in Example 10. The overall yield of human insulin as judged from HPLC was 48%. The other products were identified as Lys-Ao-insulin (42%), Des-B30-insulin (9%) and minor unidentified components (1%).

Example 13

Characterization of human insulin prepared from B-Lys-Arg-A

Human insulin was prepared as described in example 11 and characterized in the following analysis:
a) The human insulin shows only one band on basic disc electrophoresis in urea-containing polyacrylamide gels (Method described by Schlichtkrull et al., Horm.Metabol.Res., Suppl. Ser. 5 (1974) 134-143). The band migrated as pancreatic human insulin.
b) Regular shaped rhombohedral crystals were obtained by crystallization of the human insulin in the presence of Zn$^{++}$ (Method described by Schlichtkrull, Acta Chem.Scand. 10 (1956) 1459-1464).
c) The biological activity of the human insulin was determined in a mouse blood sugar depletion test. The estimated potency was 28.1 I.U./mg (p 0.05 confidens limits: 25.7-30.7 I.U./mg) using the 4th International Standard for insulin.
d) The amino acid composition of the human insulin was determined after hydrolysis at 110° C for 24 h, 48 h and 96 h in 6N HCl and the values for Thr, Ser, Pro and NH$_3$ were determined by linear regression (t = 0). The values for Val and Ile were extrapolated to indefinite time of hydrolysis. The value for 1/2 Cys was determined after hydrolysis for 24 h in 4M methansulfonic acid. The amino acid composition is given in Table 8.

Table 8

Amino acid composition of human insulin prepared from B-Lys-Arg-A

| Amino acid | Amino acid residue/molecule | |
|---|---|---|
| | Found | Theory |
| Asx | 2.95 | 3 |
| Thr | 2.90 | 3 |
| Ser | 3.00 | 3 |
| Glx | 6.95 | 7 |
| Pro | 1.06 | 1 |
| Gly | 3.95 | 4 |
| Ala | 1.04 | 1 |
| 1/2 Cys | 5.65 | 6 |
| Val | 4.10 | 4 |
| Ile | 2.00 | 2 |
| Leu | 6.00 | 6 |
| Tyr | 3.94 | 4 |
| Phe | 2.93 | 3 |
| Lys | 1.01 | 1 |
| His | 1.96 | 2 |
| Arg | 1.02 | 1 |
| $(NH_3)$ | (5.60) | (6) |

Claims

1. DNA-sequence encoding a human insulin precursor of the formula

   B-X-Y-A    (I)

   wherein B and A are the B- and A-chain of human insulin crosslinked as in human insulin and wherein X and Y are each lysine or arginine.

2. Replicable expression vehicle capable of expressing a DNA-sequence according to claim 1 in yeast.

3. A method of producing human insulin precursors of the formula

   B-X-Y-A    (I)

   in which B and A are the B- and A-chain of human insulin crosslinked as in human insulin and X and Y are each lysine or arginine, wherein a yeast strain transformed with an expression vehicle comprising a DNA-sequence encoding the insulin precursor is cultivated in a suitable culture medium and the insulin precursor is recovered from the culture medium.

4. A method for producing human insulin wherein a yeast strain transformed with an expression vehicle comprising a DNA-sequence encoding an insulin precursor of the formula

   B-X-Y-A    (I)

in which B and A are the B- and A-chain of human insulin crosslinked as in human insulin and X and Y are each lysine or arginine, is cultivated in a suitable culture medium and the insulin precursor is recovered from the culture medium and converted into human insulin by enzymatic treatment.

5.  A method according to claim 4, wherein the insulin precursor of the formula

B-X-Y-A      (I)

in which B and A are the B- and A-chains of human insulin crosslinked as in human insulin and X and Y are each a lysine or arginine, is converted into human insulin by treating an aqueous solution of the insulin precursor with trypsin and carboxypeptidase B, and thereafter recovering the human insulin from the solution.

6.  A method according to claim 5 further comprising a one-step dual enzyme treatment with trypsin and carboxypeptidase B.

7.  A method according to claim 5 further comprising treating the insulin precursor with trypsin and thereafter treating the reaction product with carboxypeptidase B.

8.  A method according to claim 7 further comprising conducting the trypsin treatment at a pH in the range of pH 11-12.

**Revendications**

1.  Séquence ADN codant pour un précurseur d'insuline humaine de la formule

B-X-Y-A      (I)

dans laquelle B et A constituent la chaîne B et A de l'insuline humaine réticulée comme dans l'insuline humaine et dans laquelle X et Y représentent chacun la lysine ou l'arginine.

2.  Véhicule d'expression réplicable pouvant exprimer une séquence ADN selon le revendication 1 dans la levure.

3.  Procédé destiné à produire des précurseurs d'insuline humaine de la formule

B-X-Y-A      (I)

dans laquelle B et A constituent la chaîne B et A de l'insuline humaine réticulée comme dans l'insuline humaine et X et Y représentent chacun la lysine ou l'arginine, où une souche de levure transformée par un véhicule d'expression comprenant une séquence ADN codant pour le précurseur d'insuline est mise en culture dans un milieu de culture approprié et le précurseur d'insuline est récupéré à partir du milieu de culture.

4.  Procédé pour la production d'insuline humaine, dans lequel la souche de levure transformée par un véhicule d'expression comprenant une séquence ADN codant pour le précurseur d'insuline de la formule

B-X-Y-A      (I)

dans laquelle B et A constituent la chaîne B et A de l'insuline humaine réticulée comme dans l'insuline humaine et X et Y représentent chacun le lysine ou l'arginine, est mise en culture dans un milieu de culture approprié et le précurseur d'insuline est récupéré à partir du milieu de culture et converti en insuline humaine par traitement enzymatique.

5.  Procédé selon la revendication 4, dans lequel le précurseur d'insuline de la formule

B-X-Y-A      (I)

dans laquelle B et A sont les chaînes B et A de l'insuline humaine réticulée comme dans l'insuline humaine et X et Y représentent chacun la lysine ou l'arginine, est converti en insuline humaine par traitement d'une solution aqueuse du précurseur d'insuline avec la trypsine et la carboxypeptidase B, et ensuite en récupérant l'insuline humaine à partir de la solution.

6. Procédé selon la revendication 5, comprenant de plus un traitement enzymatique double en une seule étape avec la trypsine et la carboxypeptidase B.

7. Procédé selon la revendication 6, comprenant de plus le traitement du précurseur d'insuline avec la trypsine et ensuite le traitement du produit réactionnel avec la carboxypeptidase B.

8. Procédé Selon la revendication 7, comprenant de plus la conduite du traitement à la trypsine à une valeur de pH située dans la plage de pH 11-12.

**Patentansprüche**

1. DNA-Sequenz, die für einen Humaninsulin-Vorläufer der Formel

B-X-Y-A     (I)

codiert, wobei B und A die B- und A-Kette von Humaninsulin sind, verknüpft wie in Humaninsulin, und X und Y jeweils Lysin oder Arginin sind.

2. Replizierbares Expressionsvehikel, das in der Lage ist, eine DNA-Sequenz nach Anspruch 1 in Hefe zu exprimieren.

3. Verfahren zur Herstellung von Humaninsulin-Vorläufern der Formel

B-X-Y-A     (I)

in der B und A die B- und A-Kette von Humaninsulin sind, verknüpft wie in Humanisulin, und X und Y jeweils Lysin oder Arginin sind, bei dem ein Hefestamm, der mit einem Expressionsvehikel transformiert ist, das eine DNA-Sequenz umfaßt, die den Insulin-Vorläufer codiert, in einem geeigneten Kulturmedium kultiviert und der Insulin-Vorläufer aus dem Kulturmedium gewonnen wird.

4. Verfahren zur Herstellung von Humanisulin, bei dem ein Hefestamm, der mit einem Expressionsvehikel transformiert ist, das eine DNA-Sequenz enthält, die einen Insulin-Vorläufer der Formel

B-X-Y-A     (I)

codiert, in der B und A die B- und A-Kette von Humanisulin sind, verknüpft wie in Humanisulin, und X und Y jeweils Lysin oder Arginin sind, in einem geeigneten Kulturmedium kultiviert und der Insulin-Vorläufer aus dem Kulturmedium gewonnen und durch enzymatische Behandlung zu Humaninsulin umgewandelt wird.

5. Verfahren nach Anspruch 4, dadurch gekennzeichnet, daß ein Insulin-Vorläufer der Formel

B-X-Y-A     (I)

in der B und A die B- und A-Kette von Humaninsulin sind, verknüpft wie in Humanisulin, und X und Y jeweils Lysin oder Arginin sind, dadurch zu Humaninsulin umgewandelt wird, daß einen wässrige Lösung des Insulin-Vorläufers mit Trypsin und Carboxypeptidase B behandelt und danach das Humanisulin aus der Lösung gewonnen wird.

6. Verfahren nach Anspruch 5, weiter gekennzeichnet durch eine einstufige duale Enzymbehandlung mit Trypsin und Carboxypeptidase B.

7. Verfahren nach Anspruch 5, weiter gekennzeichnet durch das Behandeln des Insulin-Vorläufers mit Trypsin und das anschließende Behandeln des Reaktionsproduktes mit Carboxypeptidase B.

8. Verfahren nach Anspruch 7, weiter gekennzeichnet durch das Durchführen der Trypsinbehandlung bei einem pH im Bereich von pH 11-12.

# Fig.1

Fig. 2

# Fig.3

EP 0 195 691 B1

# Fig.4

# Fig.5

Fig.6

# Fig.7

Fig. 8